# EUROPEAN PATENT APPLICATION

(11) **EP 2 368 590 A1**
(43) Date of publication of application: **28.09.2011**
(21) Application number: 11159785.2
(22) Date of filing: 25.03.2011
(51) Int. Cl.: A61M 3/00, G01F 11/02

(54) **System for dosing**

(30) Priority: 26.03.2010 BE 201000191
(71) Applicant: Pilipili Produktontwerp bvba, 8500 Kortrijk (BE)
(72) Inventor: DEHOLLANDER, Steven, 8500, KORTRIJK (BE)
(74) Representative: Ostyn, Frans

(57) **Abstract**

The invention relates to a system for dosing for delivering a repeated fixed and accurate dosage, consisting of a housing (1, 4) comprising a spring element (3) which acts on a dosing element (2), and an opening (11) in the housing (1, 4) where the dosing element (2) crosses the housing (1, 4), in which the dosing element (2) is stored in said housing (1, 4) in the rolled-up or folded-up position. The invention furthermore relates to a method for dosing using such a dosing element and the use thereof.

## Description

The invention relates to a system for dosing for a repeated fixed and accurate dosage of for example medication. The system for dosing furthermore relates to a device and a method for administering a repeated fixed dosage, in which a number of small movements can bridge a great administering distance.

A system for dosing which is commonly used today is a syringe having a dispersion opening on one side and a plunger on the other side. By displacing the plunger over a certain distance, for example up to a grade mark, a dosed amount of substance is pushed out of the syringe. The problem in this case is that the dose is possibly too small or too large, since the displacement is freely determined by the user.

In another system for dosing which enables better dosing, the plunger is moved by means of a rotary movement instead of a pushing movement. In this case, for example, a complete rotation may provide a certain dosage unit. Such a system is used, for example, for administering a dosed amount of cream in the mouth. However, it is still a drawback that the application of the rotary movement requires a certain degree of skill, on the one hand because the rotary movement has to be carried out in the longitudinal direction of the system for dosing and on the other hand because a rotary movement comprising a full turn is difficult to accomplish in one single movement. In addition, it is still possible to slightly overdose or underdose. Another drawback is the relatively complex structure of such a system for dosing compared to the prior one.

Existing systems for dosing are also designed in the form of an elongate shape of a syringe or a pen, in which a plunger/push rod is arranged in the longitudinal direction of the syringe or pen. In the starting position, the plunger then has to be extended over its entire length. This has the drawback that the system for dosing has a certain size or length.

It is therefore an object of the present invention to offer a solution for the drawbacks of existing systems. In this case, it is specifically an object to provide a system for dosing which can dose accurately by means of a simple movement, is easy to use, and is simple and inexpensive to produce. In addition, the system for dosing of the invention is very small in size, irrespective of the size of the syringe or pen in which the substance is contained.

The object of the invention is achieved by providing a system for dosing consisting of a housing comprising a spring element which acts on a dosing element, and an opening where the dosing element passes through the housing, in which the dosing element can be moved through the opening in stages and the spring element is actuated by a translational movement so that the dosing element travels a discrete distance for each translational movement.

### Summary of the invention

The invention provides a system for dosing for delivering a repeated fixed and accurate dosage, consisting of a housing comprising a spring element which acts on a dosing element, and an opening in the housing where the dosing element crosses the housing, in which, upon being pushed in, the spring element causes an extension which moves the dosing element through the opening of the housing over a discrete distance, in which the displacement of the dosing element over said discrete distance is identical each time the spring element is pushed in, and the spring element returns to a relaxed position after having been pushed in and the dosing element remains in the displaced position, in which the dosing element is stored in said housing in a rolled-up or folded-up position.

The dosing element can be moved in a longitudinal direction and the spring element is pushed in in a direction transverse to this longitudinal direction.

The dosing element preferably delivers one dosage unit for each pushing movement of the spring element.

In a preferred embodiment, the dosing element comprises indentations or protuberances with which the spring element can engage by means of a clamping action. In an alternative embodiment, the dosing element has a flat surface in which case the spring element can engage by means of frictional force.

In a particular embodiment of a dosing element according to the invention, the spring element is a leaf spring and the dosing element a rolled-up toothed strip, in which the leaf spring is attached to the housing on one side and acts on the toothed strip on the other side, in which the leaf spring acts on a tooth of the toothed strip with each pushing movement, and in which the teeth are placed in such a manner that the distance between the teeth is equal to the discrete distance which the dosing element travels. The housing comprises an upper shell and a lower shell in which the lower shell contains means for supporting the rolled-up toothed strip and attaching the leaf spring, and in which the upper shell is provided with a flexible part for operating the leaf spring. Preferably, a cavity is provided in the lower shell at the location of a protuberance which is provided in the upper shell for engaging with a tooth, in such a manner that there is sufficient space for the rolled-up toothed strip to move past the entire protuberance.

In a particular embodiment, the spring element completely or partly forms part of the housing.

The spring element is preferably a metal or plastic formed or bent element, made from stainless steel, POM or PA.

The dosing element and the housing are preferably made from stainless steel, polypropylene, polyacetal, POM, LDPE or HDPE.

In an advantageous embodiment, the system for dosing is a disposable system.

In an alternative embodiment, the system for dosing can operate in two directions, both in the direction out of and into the housing.

In a second aspect, the invention provides a method for delivering a repeated fixed and accurate dosage by means of a system for dosing according to one of the preceding claims, comprising the following steps:
- Step a: system for dosing with a flexible strip which is initially completely rolled up in the housing and provided with a container filled with substance to be metered,
- Step b: placing the system for dosing in the palm of a hand so that the control mechanism of the spring is at the top of the thumb and the container with substance is situated in the space between the fingers and thumb,
- Step c: pushing in the leaf spring using the thumb, so that the leaf spring engages with a tooth of a rolled-up flexible strip, as a result of which the latter is moved out of the housing into the container over the distance of a tooth, and thus delivers a fixed dose of substance via the open end of the container,
- Step d: releasing the leaf spring so that it no longer engages with the strip and returns to its original relaxed position, in which the rolled-up strip remains in the displaced position by being clamped to the tooth.

In yet another aspect, the invention provides the use of a system for dosing as described here for delivering or taking up a dosed amount of solid or liquid matter, such as cream, insulin, pills, tablets, peppermints, silicone, solder.

### Description of the invention

The invention is based on the principle that a substantially bent or shaped spring element which is situated, for example, on a horizontal surface and on which pressure is exerted, becomes flatter and causes an extension in the horizontal plane. If one side of the spring element is attached to the surface, then the other side will experience a displacement in the plane. Said displacement can be used to move another element, for example the dosing element. Once it is outside the housing, the dosing element has the function and characteristics of a plunger.

The housing can entirely or partly enclose the system for dosing and/or the spring element, with the intention to offer sufficient support to both. In another embodiment, the spring element partly or completely forms part of the housing.

The dosing element, which is partly or completely enclosed in the housing, leaves/enters the housing via an opening.

The housing furthermore provides an opening for the operation or control of the spring element.

The spring element acts on the dosing element in order to provide a certain dosage/step/distance of the dosing element. This can take place in two ways: either the dosing element provides these steps by, for example, the toothed indentations, or the spring element provides these steps by, for example, a fixed displacement each time it is pressed.

The dosing element can be moved stepwise in the housing. In this case, one or several steps are directly related to the dosage unit. In a particular embodiment, the dosing element is moved by the same distance for each step. The dosing element preferably provides that one step equals one dosage unit.

In a preferred embodiment, the system for dosing is configured in such a manner that it has identical steps. In another embodiment, the system for dosing may serve for a therapy for certain days, for example, in which the last steps/dosages are smaller than the first. Such a gradual dosage can be provided by a system for dosing comprising gradual steps. In another embodiment, the invention provides systems for dosing of different sizes of steps, so that different dosages can be administered, depending on the system for dosing used. To this end, the invention provides dosing elements having different step sizes, or spring elements which produce different dosages.

The term translational movement is to be understood as a rectilinear or linear movement in a specific direction, in contrast with a rotary movement. In a particular embodiment, the dosing element is movable in a longitudinal direction and the spring element is operated in a direction transversely to this longitudinal direction. In another embodiment, the spring element may provide a translational movement in either the longitudinal direction or at any angle with respect to the direction of movement of the dosing element.

For each translational movement of the spring element, the dosing element travels a certain discrete distance (or step). In a particular embodiment, the dosing element itself determines the distance (or step). In another embodiment, the distance (or step) is determined by (the freedom of movement of) the spring element. Dosing is carried out in one or more discrete steps.

In one direction of the translational movement, the spring element acts on the dosing element, as a result of which the latter is displaced; in the other direction, the spring element does not act on the dosing element and preferably does not displace the latter.

The spring element is an element which causes an extension when it becomes flatter after having been pushed. As the spring element acts on the dosing element, this extension produces a displacement of the dosing element. This displacement is preferably constant for each new pressing movement. If desired, even the extension may be constant.

In a particular embodiment, the translational movement comprises an in/out movement, such as for example of a push-button. In a more particular embodiment of the system for dosing of the invention, the translational movement of the spring element is transferred onto the dosing element by means of a leaf spring which acts on the dosing element, with a pushing movement of the leaf spring causing a discrete movement of the dosing element as a result of the translational movement.

Spring elements are for example metal or plastic shaped or bent elements, made of for example stainless steel, POM or PA plastic.

The length of the spring depends on the housing and may be larger or smaller than the latter, depending on the location where the spring engages with the strip. The length of the rolled-up strip only depends on the size of the housing. Another form of housing is possible.

In another embodiment, the spring element forms part of the housing. In one particular embodiment, the upper shell is the spring element. In another embodiment, both the upper and the lower shell form part of the spring element, or the upper and lower shell are designed such that they form one shell or one spring element.

In a particular embodiment, a clicking sound is produced during dosing. This clicking sound may be produced by any element of the system for dosing during the dosing movement.

The dosing element is preferably any element which can be stored in the housing in either an arbitrary manner, such as for example a wire, or in a specific folded manner, such as for example rolled up or folded up. What is typical in this case, is that a large extension can be produced by the dosing element, while the dosing element itself fits into the small housing. In this case, it is important that the dosing element can be removed from and moved into the housing in an efficient manner by the spring element and does not become entangled. In a particular embodiment, the dosing element of the invention could, for example, contain a roll of fabric having a width of several metres.

In a further embodiment, the dosing element comprises a roll-up element on which a spring element, preferably a leaf spring, can act in order to displace this element over a discrete distance with respect to the housing.

In an advantageous embodiment, the roll-up element contains indentations or protuberances, for example in the form of teeth, which the spring element can grip. In another embodiment, the dosing element is, for example, a wire with a smooth surface (without teeth) and has a sufficiently great friction/hardness for the spring element to be able to grasp it due to a sufficient frictional force. The discrete movement of the spring element then ensures a discrete movement of the "wire". In yet another embodiment, the difference in hardness between the spring element and the dosing element at the location of action ensures an efficient interaction. This may be the case, for example, if a relatively hard element engages with and presses on the surface of a relatively soft element.

In yet another embodiment, the spring element may be provided, at the point of contact with the dosing element, with a third element which promotes the interaction between the two elements, for example, an improved engagement, friction or difference in hardness.

In its most advantageous embodiment, the dosing element comprises a toothed strip which is rolled up in the housing, in which a leaf spring acts on a tooth by a translational or pushing movement on a tooth, and in which the teeth are positioned in such a manner that the distance between the teeth is equal to the discrete distance which the dosing element travels.

The system for dosing is preferably provided with a stop system to prevent the toothed strip of the dosing element from moving back after the leaf spring is released. The system for dosing is also provided with a stop system to control the maximum extension of the spring element.

The system for dosing is particularly advantageous in the sense that the size of the housing is not limited by the maximum length to be bridged by the dosing element, as is usually the case with conventional syringe systems for dosing. The housing of the system for dosing is only limited by the size of the spring element and the stored dosing element.

In a particular embodiment, the dosing element is stored in the housing in its entirety before dosing is started, with the length of the dosing element not being dependent on the length of the housing.

In a particular embodiment, the housing of the system for dosing comprises an upper and a lower shell, with the lower shell containing means to support the rolled-up strip and to attach the leaf spring in such a manner that the leaf spring acts on the rolled-up strip, and with the lower and the upper shell being releasably attached to one another by means of attachment means.

The element which form part of the system for dosing, the spring element, the dosing element and the housing, are preferably made of stainless steel, polypropylene, polyacetal, POM, LDPE, HDPE or other materials which are suitable for the purpose. The material of the dosing element is preferably sufficiently flexible to be rolled up, but not too flexible so that it does not fold when it is pushed into the container and has sufficient stiffness. In such a case, the system for dosing may be provided with additional means which support the dosing element.

The invention furthermore provides a system for metered dispersion of a substance, comprising a system for dosing as described above and a coupling system for the releasable attachment of the opening of the housing to a container containing the substance, in which the dosing element in the container moves through the opening in order to disperse the substance in a metered way from an opening in the container. In a particular embodiment, the container is in the shape of a cylinder, such as a syringe. Other shapes of a container are also possible and may be dependent on the substance to be metered.

Depending on the kind of substance to be metered and the amount to be metered, the cylinder diameter and the opening may be relatively large or relatively small. The dosing element preferably has a circular end which slidably fits into the releasably attached container and ensures that the substance in the container is advanced in an efficient manner. This end then also serves as a plunger for the dispersion. The plunger is blocked by an element of the housing, so that the dosing element cannot move back beyond this point in the housing. This also provides a start position of the dosing element.

In another embodiment, this end does not serve as a plunger as such, but can be coupled to a plunger which forms part of a container set (container and matching plunger). This has the advantage that the plunger and container are perfectly matched and can be produced as such in order to be coupled to a system for dosing of the invention. Another advantage is the fact that the system for dosing does not pose any limitations on the kind of container (diameter, shape, etc.) which can be used, and that the system for dosing can be used for any container set which can be coupled thereto. To this end, the housing will allow such a container set to be coupled, optionally with a connecting piece on the opening of the housing in order to allow for different diameters.

In another embodiment, the system for dosing of the invention provides a system for dosing which can also be used for metered sucking up or pulling up, being the reverse movement of that which is required for dispersion. To this end, the system for dosing may be provided with a pulling system, in which the spring element pulls the dosing element into the housing in steps.

In a preferred embodiment, the system for dosing is a disposable system. This is made possible in order to be able to use inexpensive materials and a simple and inexpensive design. Other advantages of the system for dosing of the invention are (a) the small, compact dimensions, irrespective of the size of the container, (b) the use of fine pens or containers as a result of using fine dosing elements or rolled-up strips, (c) the bridging of great distances, (d) an accurate (discrete, precise and repetitive) dosage as the dosing step is very discrete, (e) a fine adjustment or dosage, and (f) convenient and simple operation by means of translational or push-button movement. Finally, the system for dosing may be designed to be able to operate in two directions (into and/or out of the housing).

The invention furthermore provides a method for repeated accurate dosage using a single pushing movement, the method comprising the following steps:
- Step a: pushing the spring element of a system for dosing of the invention resulting in an extension of the spring ends causing a displacement of the dosing element over a fixed distance with respect to the housing,
- Step b: fixing the dosing element in the displaced position, and
- Step c: releasing the spring element so that it no longer engages with the dosing element and returns to its original position.

In a particular embodiment, the method comprises the following steps:
- Step a: the system for dosing with the flexible strip initially completely rolled up in the housing and provided with a container filled with substance to be metered,
- Step b: placing the system for dosing in the palm of a hand so that the control mechanism of the spring is at the top of the thumb and the container with substance is situated in the space between the fingers and thumb,
- Step c: pushing in the leaf spring using the thumb, so that the leaf spring engages with a tooth of a rolled-up flexible strip, as a result of which the latter is moved out of the housing into the container over the distance of a tooth, and thus delivers a fixed dose of substance via the open end of the container,
- Step d: releasing the leaf spring so that it no longer engages with the strip and returns to its original relaxed position, in which the rolled-up strip remains in the displaced position by being clamped to the tooth.

If desired, it is possible to first ensure that the substance is situated completely at the open end of the container by initially performing a number of pushing movements and dosages, as is also the case when initially removing air from a syringe.

The invention furthermore provides the use of a system for dosing as described above for administering a metered amount of food or medication, either in liquid or solid form. Liquid forms are, for example, creams or injection liquids. Solid forms are, for example, pills or tablets of either medication or, for example, sweets or mints which are pushed out or metered per tablet.

In another embodiment, the use of a system for dosing is provided for sucking up, pulling up or removing a metered amount of, for example, liquid.

In a specific example, the invention relates to a system for dosing for applying a cream against aphthous ulcers. In another example, the invention relates to a system for dosing for injecting a certain amount of medication. In yet another example, the invention relates to a system for dosing or pipetting system for metered pipetting.

In another area of application, and in a stronger format, the system for dosing of the invention can be used for metering silicone or solder.

In another particular example, the invention relates to a system for dosing for sucking up a certain amount of liquid material, or stepwise sucking up of a material, for example germ or sperm cells or eggs. To this end, preferably a suitable container/sucker is used which is coupled to the system for dosing of the invention. In yet another application, the invention relates to a system for dosing for performing an injection for diabetes, for example, in which the dosage step serves to insert a needle from a container into the skin for a certain length/depth and to inject the insulin medication. To this end, a needle set may be used as an addition to the container set described above in which the needle is moved with respect to the dosing element via coupled ends.

In a typical embodiment, the dosing element is displaceable in the direction moving away from the housing. In another embodiment, the dosing element is displaceable in the direction moving towards the housing.

The housing may comprise one or more dosing elements, displaceable in the same direction or not, via the same opening or not. The dosing elements may be provided with the same or different dosage steps.

### Description of the figures

The invention will now be described in more detail by means of the following detailed description of a preferred spreading device according to the invention. The sole aim of this description is to give an illustrative example and to indicate further advantages and features of the present invention and can therefore by no means be interpreted as a limitation of the area of application of the invention or of the patent rights defined in the claims.

In this detailed description, reference numerals are used to refer to the attached drawings, in which:
- Fig. 1 shows an exploded view of a system for dosing according to the invention;
- Fig. 2 shows an assembled system for dosing from Fig. 1 (a) without and (b) with an upper shell, having an opening for a flexible part;
- Fig. 3 shows a bottom view of the upper shell of the system for dosing from Fig. 1;
- Fig. 4 shows a cross section of a system for dosing according to the invention, with (a) a released and (b) a pushed-in spring tooth;
- Fig. 5 shows a system for dosing according to the invention with the dosing strip completely extended;
- Fig. 6 shows a top view and a side view of a system for dosing according to the invention with a connected container;
- Fig. 7 shows possible embodiments of a flexible strip on which a leaf spring or other spring element can act;
- Fig. 8 shows an alternative system for dosing according to the invention; and
- Fig. 9 shows an alternative form of a spring element.

A preferred embodiment of a system for dosing according to the invention consists of a housing which comprises a leaf spring and a rolled-up toothed strip, in which one side of the leaf spring is fixed to the housing and the other side acts on the strip, in which the leaf spring can be pushed in by means of a flexible control mechanism at the top of the housing, and the strip with a tooth is pushed forwards, out of the housing in a longitudinal direction as a result of a pushing movement, in such a manner that the strip is fixed in this displaced position by means of the tooth after the leaf spring returns to its original position, and the toothed strip moves over the fixed distance of a tooth at each pushing movement of the leaf spring.

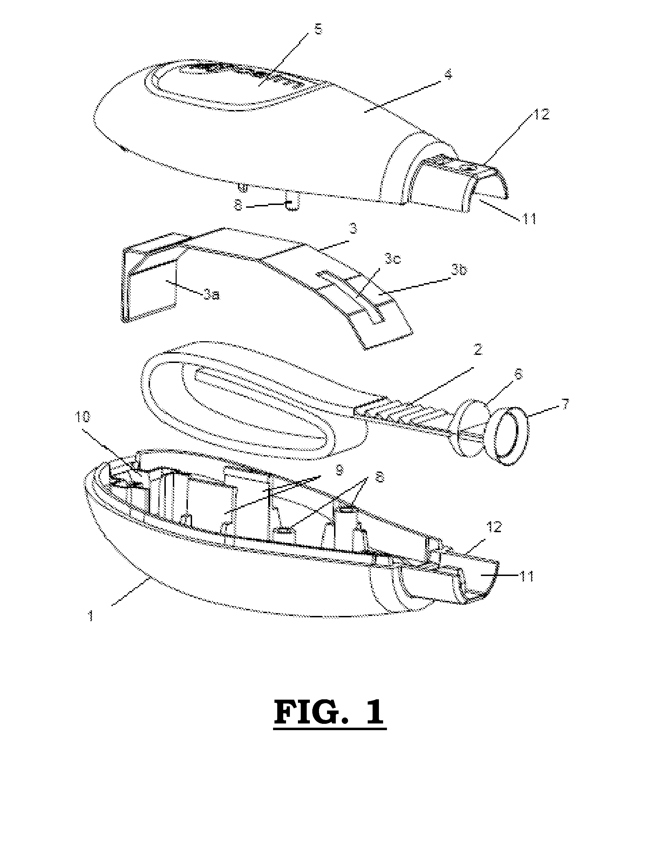

Such a system for dosing is represented in Fig. 1 and consists of a lower shell (1), a rolled-up flexible strip (2) with small teeth along virtually its entire length (only partly shown in the figure.), a spring (3), and an upper shell (4) with a flexible central part (5) for pushing in the spring. The flexible central part (5) preferably consists of rubber or of another material which can be pushed in.

The spring (3) has a fixed (3a) and a flexible side (3b), the latter serves to engage with the strip. Here, the leaf spring (3) is optionally designed to have an opening (3c) on the flexible side. The housing is provided with a protruding element (17) which supports or keeps the rolled-up strip (2) flat in such a manner via this aperture that the spring (3) engages with it more effectively. Such a support (17) may also have another embodiment.

The lower shell (1) and the upper shell (4) may be releasably attached to one another by means of attachment means (8). Both shells (1, 4) have an opening (11) via which the rolled-up strip (2) can leave the housing.

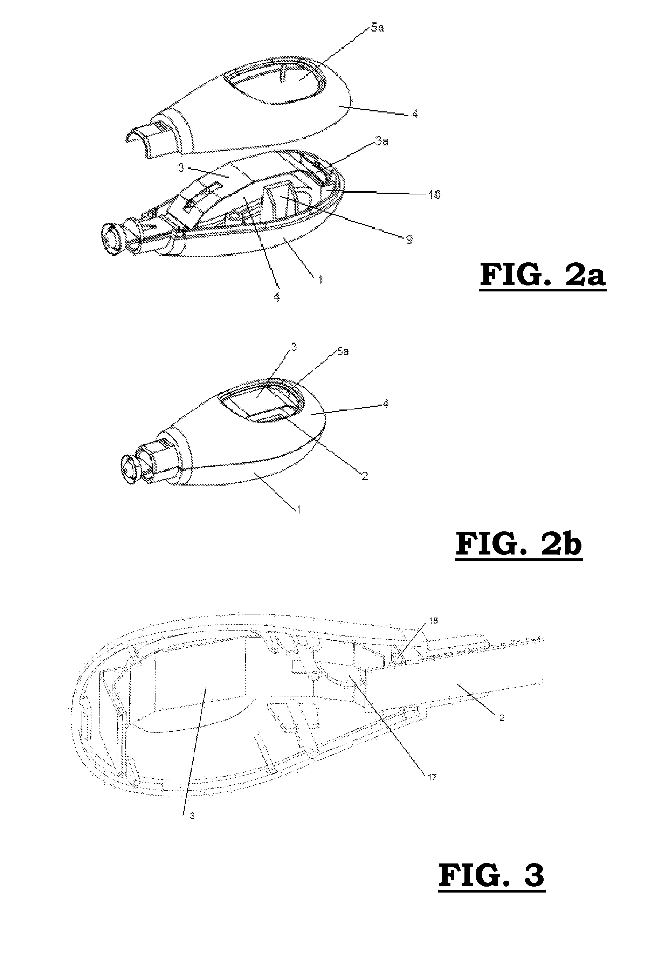

The lower shell (1) contains support means (9) for positioning and supporting the rolled-up strip and to fix the spring on one side (10), as is also shown in Fig. 2a. Other embodiments of these support means are possible. Thus, the fixed side (3a) of the spring may also be of a different embodiment.

The upper shell (4) which, like the lower shell, is made from a material which offers sufficient strength to the system for dosing, has an opening (5a) into which the flexible central part (5) for pushing in the spring fits. Fig. 2b clearly shows that the convex part of the spring (3) which can be pushed in is situated below this opening (5a).

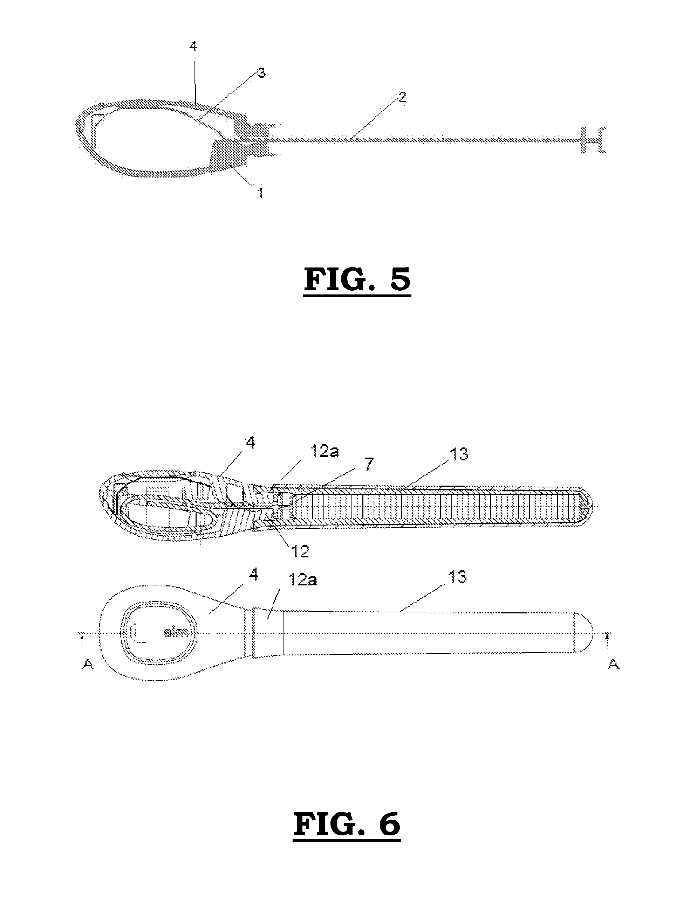

At the location of the opening (11), the housing (1, 4) is provided with a connecting piece (12) to which a container or pen (13) comprising a substance to be pushed out can be connected, as is illustrated in Fig. 6. This connecting piece (12) preferably has a diameter which matches the diameter of the connecting piece (12a) of the container or pen (13).

The rolled-up strip (2) has a plunger (7) at one end. The diameter of the plunger (7) is almost the same or slightly smaller than the inner diameter of the container or pen (13) in such a manner that the substance in the pen is pushed forward by the plunger. The protruding element (6) positions the rolled-up strip (2) in an initial position during assembly in the housing.

As is illustrated in Fig. 3, the spring (3) can carry out a maximum displacement which is determined by the stop (18) which is provided in the housing. This maximum displacement provides a dosage unit. In a particular embodiment, the stop (18) could be adjustable, so that a certain amount or dosage unit can be set beforehand.

If no force is exerted on the leaf spring (3), the latter is in a convex position (spring position in solid line). One tooth of the rolled-up strip (2) remains engaged with a protuberance (15) which forms part of the upper shell (4). This protuberance (15) ensures that the strip (2) can only move out of the housing in a forward direction and does not move back into the housing together with the spring which is released. An embodiment other than this protuberance (15) is also possible to achieve the same effect.

By pushing on the spring (3) via the flexible central part (5), the spring will become flatter (spring position in broken line), and the spring will engage with a tooth of the rolled-up strip (2). As a result thereof, the strip (2) will be pushed forward by a tooth. In this way, the rolled-up strip (2) can be moved out of the housing completely, tooth by tooth, pushing movement by pushing movement.

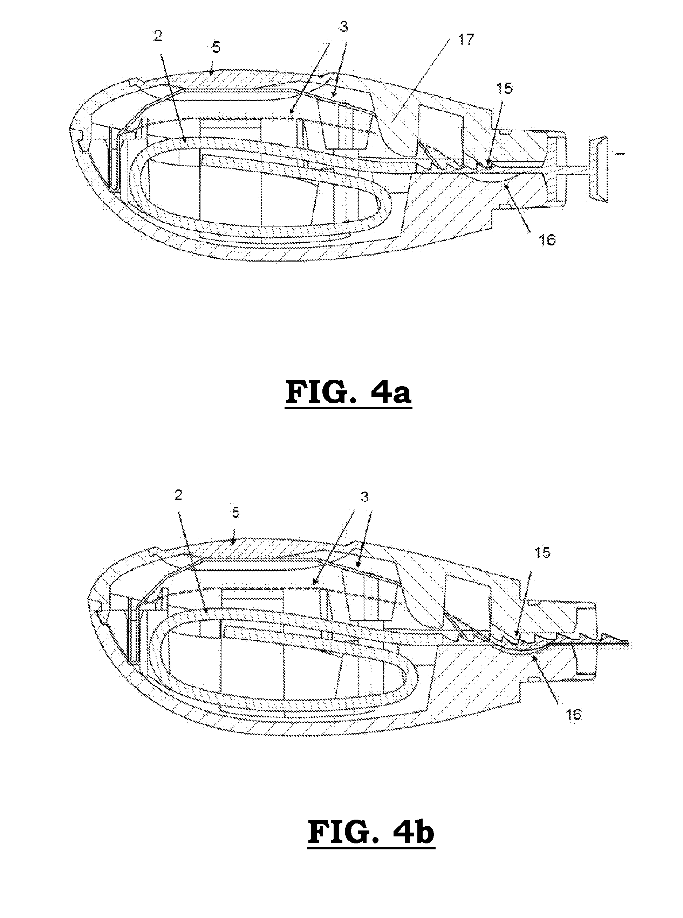

A cavity (16) provided in the lower shell (1) at the location of the protuberance (15) ensures that there is sufficient space for a tooth on the strip to be moved past the protuberance (15), as is illustrated in the detail of Fig. 4b.

Fig. 5 shows a system for dosing with a completely extended and unrolled strip (2). In this case, it is clear that the dimensions of the housing (1, 4) can be very compact and are independent from the length of the strip (2) or the attached pen (13). The attached pen (13) from Fig. 6 then has a maximum length which can be covered by the entire length of the unrolled strip (2).

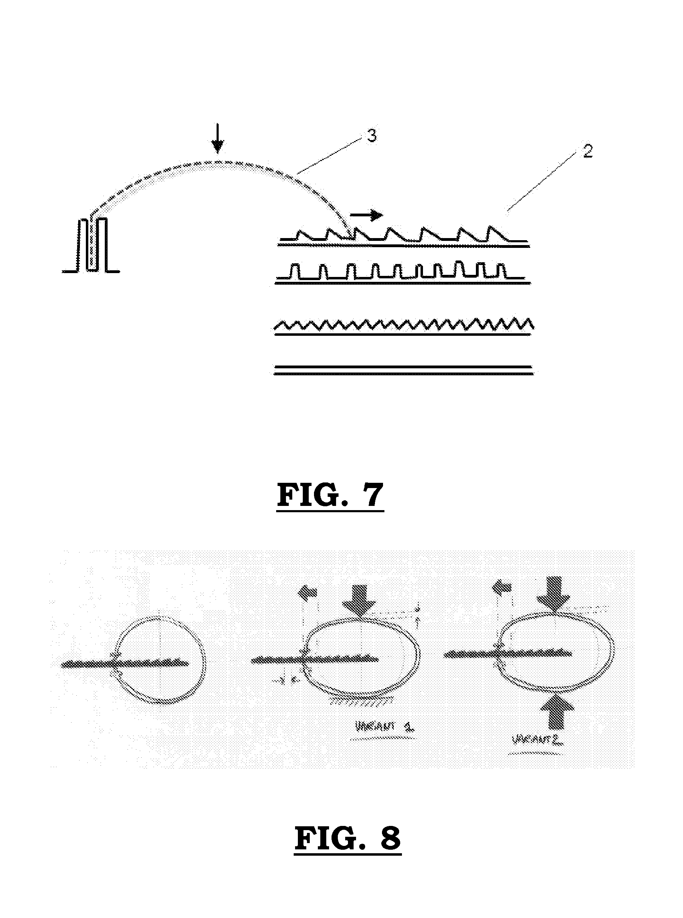

Fig. 7 shows some possible embodiments of a strip (2) with differently shaped indentations, for example toothed, rectangular or triangular and flat.

Fig. 8 shows an alternative embodiment of a system for dosing according to the invention in which the spring element can, for example, also form part of the housing. The convex shape of the leaf spring produces a force which, in a variant form, can be used both on one side and on two sides (at the top and at the bottom).

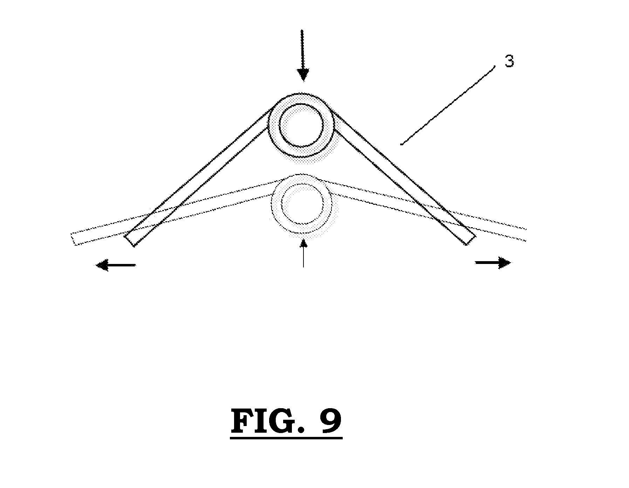

Fig. 9 shows an alternative to the leaf spring (3). Pushing in the spring in the downward direction causes an extension between the spring ends in the horizontal direction. The extension can be used to displace a dosing element in a system for dosing according to the invention.

## Claims

1. System for dosing for delivering a repeated fixed and accurate dosage, consisting of a housing (1, 4) comprising a spring element (3) which acts on a dosing element (2), and an opening (11) in the housing (1,4) where the dosing element (2) crosses the housing (1, 4), in which:
- upon being pushed in, the spring element (3) causes an extension which moves the dosing element (2) through the opening (11) of the housing (1, 4) over a discrete distance,
- with the displacement of the dosing element (2) over said discrete distance being identical each time the spring element (3) is pushed in, and
- the spring element (3) returns to a relaxed position after having been pushed in and the dosing element (2) remains in the displaced position,
**characterized in that** the dosing element (2) is stored in said housing (1, 4) in a rolled-up or folded-up position.

2. System for dosing according to Claim 1, **characterized in that** said dosing element (2) can be moved in a longitudinal direction and the spring element (3) is pushed in in a direction transverse to this longitudinal direction.

3. System for dosing according to Claim 1 or 2, **characterized in that** the dosing element (2) delivers one dosage unit for each pushing movement of the spring element (3).

4. System for dosing according to one of Claims 1 to 3, **characterized in that** the dosing element (2) comprises indentations or protuberances with which the spring element (3) can engage by means of a clamping action.

5. System for dosing according to one of Claims 1 to 3, **characterized in that** the dosing element (2) has a flat surface in which case the spring element (3) can engage by means of frictional force.

6. System for dosing according to one of the preceding claims, **characterized in that** the spring element (3) is a leaf spring and the dosing element (2) is a rolled-up toothed strip, in which the leaf spring (3) is attached to the housing (1, 4) on one side and acts on the toothed strip (2) on the other side, in which the leaf spring (3) acts on a tooth of the toothed strip (2) with each pushing movement, and in which the teeth are placed in such a manner that the distance between the teeth is equal to the discrete distance which the dosing element (2) travels.

7. System for dosing according to Claim 6, **characterized in that** the housing comprises an upper shell (4) and a lower shell (1), in which the lower shell (1) contains means for supporting the rolled-up toothed strip (2) and attaching the leaf spring (3), and in which the upper shell (4) is provided with a flexible part (5) for operating the leaf spring (3).

8. System for dosing according to Claim 6 or 7, **characterized in that** a cavity (16) is provided in the lower shell (1) at the location of a protuberance (15) which is provided in the upper shell (4) for engaging with a tooth, in such a manner that there is sufficient space for the rolled-up toothed strip (2) to move past the entire protuberance (15).

9. System for dosing according to one of the preceding claims, **characterized in that** the spring element (3) completely or partly forms part of the housing (1, 4).

10. System for dosing according to one of the preceding claims, **characterized in that** the spring element (3) is a metal or plastic formed or bent element, made from stainless steel, POM or PA.

11. System for dosing according to one of the preceding claims, **characterized in that** the dosing element (2) and the housing (1, 4) are made from stainless steel, polypropylene, polyacetal, POM, LDPE or HDPE .

12. System for dosing according to one of the preceding claims, **characterized in that** the system for dosing is a disposable system.

13. System for dosing according to one of the preceding claims, **characterized in that** the system for dosing can operate in two directions, both in the direction out of and into the housing (1, 4).

14. Method for delivering a repeated fixed and accurate dosage by means of a system for dosing according to one of the preceding claims, **characterized in that** the method comprises the following steps:
- Step a: a system for dosing with a flexible strip (2) which is initially completely rolled up in the housing (1, 4) and provided with a container (13) filled with substance to be metered,
- Step b: placing the system for dosing in the palm of a hand so that the control mechanism (5) of the spring (3) is at the top of the thumb and the container (13) with substance is situated in the space between the fingers and thumb,
- Step c: pushing in the leaf spring (3) using the thumb, so that the leaf spring (3) engages with a tooth of a rolled-up flexible strip (2) as a result of which the latter is moved out of the housing (1, 4) into the container (13) over the distance of a tooth, and thus delivers a fixed dose of substance via the open end of the container,
- Step d: releasing the leaf spring (3) so that it no longer engages with the strip (2) and returns to its original relaxed position, in which the rolled-up strip (2) remains in the displaced position by being clamped to the tooth.

15. Use of a system for dosing according to one of Claims 1 to 13 for delivering or taking up a dosed amount of solid or liquid matter, such as cream, insulin, pills, tablets, peppermints, silicone, solder.
